# EUROPÄISCHE PATENTSCHRIFT

(11) **EP 0 727 197 B1**
(45) Veröffentlichungstag und Bekanntmachung des Hinweises auf die Patenterteilung: **29.08.2001**
(21) Anmeldenummer: 95810107.3
(22) Anmeldetag: 17.02.1995
(51) Int. Cl.: A61F 2/46, A61F 2/30

(54) **Vorrichtung zum Anbringen einer Markraumsperre im Markraum eines Röhrenknochens**
Apparatus for placing an intramedullary plug in the medullary canal of a tubular bone
Dispositif pour poser un obturateur intramédullaire dans le canal médullaire d'un os tubulaire

(43) Veröffentlichungstag der Anmeldung: 21.08.1996
(73) Patentinhaber: Sulzer Orthopädie AG, 6340 Baar (CH)
(72) Erfinder: Mittelmeier, Wolfram, Dr., D-23566 Lübeck (DE); Limacher, Urs, CH-6331 Hünenberg (CH)
(74) Vertreter: Sulzer Management AG

(56) Entgegenhaltungen:
- EP-A- 0 093 560
- EP-A- 0 178 174
- WO-A-93/08769
- DE-A- 2 311 521
- DE-A- 3 835 853
- DE-A- 3 937 786
- FR-A- 2 629 337
- FR-A- 2 708 192
- US-A- 4 399 814
- US-A- 4 488 549

## Beschreibung

Die Erfindung betrifft eine Vorrichtung zum Anbringen einer Markraumsperre im Markraum eines Röhrenknochens, der zur Aufnahme eines unter Verwendung von Knochenzement zu implantierenden Verankerungsschafts einer Endoprothese bestimmt ist.

Für den inneren Abschluss eines im Markraum eines Röhrenknochens geschaffenen, mit Knochenzement zu füllenden Operationshohlraums, in den ein Prothesenschaft eingesetzt werden soll, sind zahlreiche Ausführungen von Markraumsperren in Form von Einsatzkörpern bekannt, welche jeweils im Operationshohlraum verankert werden, wobei ein im Markraum verbleibender Pfropfen gebildet wird, der ein Abfliessen des eingebrachten Knochenzements aus dem proximalen Abschnitt in den distalen Abschnitt des Markraums verhindern soll. Eine bekannte Markraumsperre der genannten Art enthält einen durch ein Setzinstrument in den Markraum einführbaren und in diesem aufspreizbaren Einsatzkörper, der aus einem körperverträglichen und körperbeständigen Kunststoff, z.B. Polyäthylen, oder aus einem der für Endoprothesen üblichen Metalle oder Metalleqierunqen bestehen kann (EP-A 0058744).

Die Merkmale des Oberbegriffs des Anspruchs 1 sind aus der DE-A-3 937 786 (siehe Figur 7) bekannt. Bei der aus dieser Druckschrift bekannten Vorrichtung handelt es sich jedoch um die zu implantierende Endoprothese selbst.

Im Knochen verbleibende Markraumsperren haben den Nachteil, dass sie im Falle eines Prothesenwechsels aus dem Knochen entfernt werden müssen, was einen entsprechenden, unter Umständen erheblichen Aufwand erfordert. Bei derartigen Markraumsperren können ferner Probleme auftreten, wenn der Einsatzkörper z.B. am Applikationsort nicht ausreichend fest verankert ist und/oder den Markraum nicht ausreichend dicht abschliesst. Bei ungenügender Verankerung des Einsatzteils kann dieser verrutschen, so dass der vom Knochenzement auszufüllende Abschnitt des Markraums verlängert und damit die Verdichtung des Knochenzements beeinträchtigt wird. Bei ungenügender Dichtheit der Markraumsperre kann Knochenzement in den distalen Abschnitt des Markraums gelangen und/oder der im distalen Abschnitt herrschende Druck durch aus dem proximalen Abschnitt des Markraums verdrängte Luft erhöht werden, wodurch Embolien entstehen können.

Der Erfindung liegt die Aufgabe zugrunde, eine insbesondere in dieser Hinsicht verbesserte Vorrichtung der eingangs genannten Art zu schaffen, welche eine präzise Positionierung derselben im Markraum gestatten und einen sicheren, dichtenden Abschluss des Markraums gewährleisten und welche eine geringere Belastung des Röhrenknochens sowie, insbesondere im Falle eines Prothesenwechsels, einen geringeren Operationsaufwand bei der Vorbereitung des für die Aufnahme des Knochenzements bestimmten Markraums erfordern.

Diese Aufgabe wird durch die im Anspruch 1 angegebenen Merkmale gelöst.

Die erfindungsgemäss ausgebildete Vorrichtung gestattet auf einfache Weise die Anbringung eines temporär, während einer relativ kurzen Phase der Operationsdauer, in den Markraum einzusetzenden, durch Druckmittelzufuhr dichtend an die Wand des Markraums anlegbaren und durch Druckmittelabfuhr schrumpfbaren Sperrelements sowie die Entfernung des geschrumpften, in die Grundstellung zurückgeführten Sperrelements aus dem Markraum. Das Sperrelement kann bereits wenige Minuten nach dem Einbringen des Knochenzements, sobald dieser eine vorbestimmte, vom Chirurgen auf einfache Weise feststellbare knetbare Konsistenz aufweist, durch Ablassen des Druckmittels aus der Expansionsstellung in die im Bereich des Querschnitts des Führungsteils befindliche Grundstellung zurückgeführt und anschliessend mit dem Führungsteil aus dem teilweise ausgehärteten Knochenzementbett herausgezogen werden. Der beim Zurückziehen des Führungsteils entstehende Hohlraum kann durch Verdichten des Knochenzementbettes aufgefüllt werden.

Ausgestaltungen der Erfindung sind in den abhängigen Patentansprüchen angegeben.

Eine erfindungsgemäss ausgebildete Markraumsperre ist Gegenstand des Patentanspruchs 10.

Die Erfindung wird anhand von in der Zeichnung schematisch dargestellten Ausführungsbeispielen erläutert, wobei die Ausführungsbeispiele der Figuren 9 bis 11 nicht unter den Wortlaut des Anspruchs 1 fallen. Es zeigen:
- Fig.1: einen axialen Teil-Längsschnitt eines Röhrenknochens mit einer erfindungsgemässen Vorrichtung zum Anbringen einer Markraumsperre,
- Fig.2,3 und 4: je einen Querschnitt des Röhrenknochens entsprechend der Linie II - II bzw. III - III bzw. IV - IV in Fig.1,
- Fig.5 und 7: den Röhrenknochen gemäss Fig.1 mit weiteren Vorrichtungen zum Anbringen einer Markraumsperre, je nach einer abgewandelten Ausführungsform,
- Fig.6 und 8: je einen Querschnitt des Röhrenknochens entsprechend der Linie VI - VI in Fig.5 bzw. der Linie VIII - VIII in Fig.7,
- Fig.9: den Röhrenknochen gemäss Fig.1 mit einer Vorrichtung nach einer weiteren abgewandelten Ausführungsform,
- Fig.10: einen Querschnitt des Röhrenknochens entsprechend der Linie X - X in Fig.9, und
- Fig.11: eine Einzelheit einer Vorrichtung gemäss Fig. 9 in einer abgewandelten Ausführungsform.

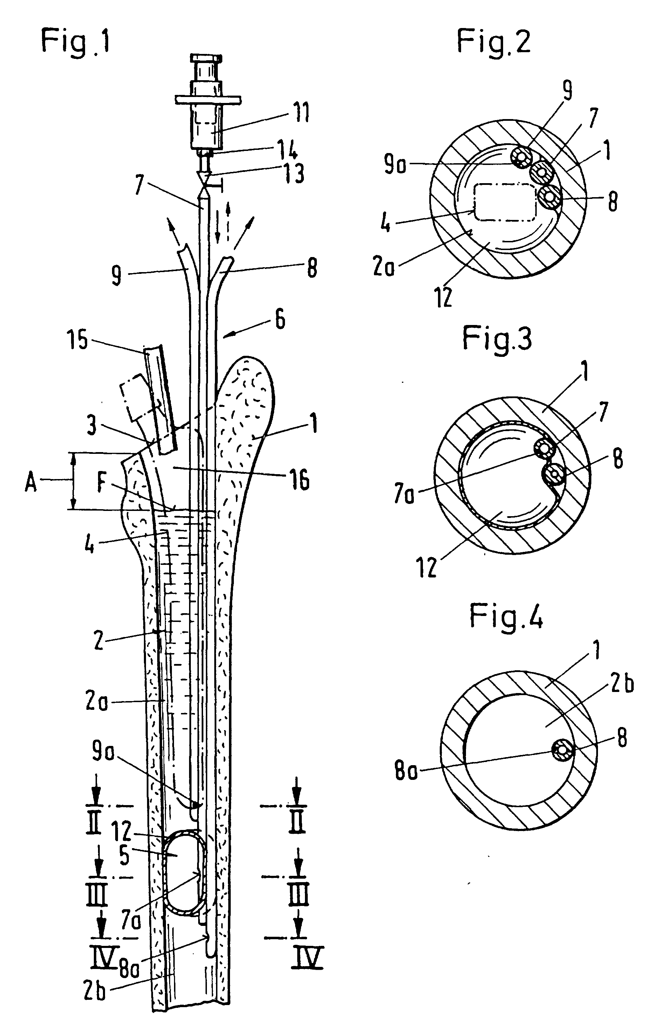

Der Röhrenknochen 1 nach Fig.1, darstellungsgemäss ein Femur, weist in seinem oberen Endabschnitt einen im Markraum 2 geschaffenen Operationshohlraum auf, der zur Aufnahme eines durch eine Operationsöffnung 3 einzusetzenden, strichpunktiert dargestellten Verankerungsschafts 4 einer Femurkopfprothese und eines den Verankerungsschaft 4 umgebenden Knochenzementbettes sowie einer Markraumsperre 5 bestimmt ist. Durch die Markraumsperre 5 wird die Eindringtiefe des Verankerungsschafts 4 begrenzt und ein mit Knochenzement zu füllender proximaler Abschnitt 2a des Markraums 2 gegen dessen distalen Abschnitt 2b abgeschlossen.

Zum Anbringen der Markraumsperre 5 ist eine in den Markraum 2 einführbare und zurückziehbare Vorrichtung 6 vorgesehen, welche einen rohrförmigen Führungsteil 7 und zwei rohrförmige Entlastungsleitungen 8 und 9 umfasst. Der Führungsteil 7 ist mit seinem ausserhalb des Markraums 2 verbleibenden Ende an eine Quelle eines Druckmittels, darstellungsgemäss eine mit einer Druckflüssigkeit, z.B. Ringerlösung, füllbaren Pumpenzylinder einer Spritze 11 anschliessbar. Der Führungsteil 7 und die Entlastungsleitungen 8 und 9, welche je durch einen aus einem gummiartigen Material, z.B. Polyäther, bestehenden flexiblen Schlauch gebildet sein können, sind an ihren in den Markraum 2 einführbaren Endabschnitten in axialer Richtung verschlossen und je mit einer seitlichen Oeffnung 7a, 8a bzw. 9a versehen. Die aus dem Markraum 2 vorstehenden Enden der Entlastungsleitungen 8 und 9 können offen oder je an eine nicht dargestellte Absaugeinrichtung angeschlossen sein.

Als Markraumsperre 5 ist ein elastisch verformbares Sperrelement in Form eines ballonartig expandierbaren, dünnwandigen Hohlkörpers 12 vorgesehen, welcher aus einem gummiartigen Material besteht und welcher nach Art des Ballons eines Blasenkatheters an dem in den Markraum 2 einführbaren Endabschnitt des Führungsteils 7 seitlich angebracht und an diesen über die Oeffnung 7a angeschlossen ist. Der Hohlkörper 12, dessen Wandstärke in der Zeichnung übertrieben gross dargestellt ist, nimmt bei druckentlastetem Führungsteil 7 eine Grundstellung ein, in welcher er sich innerhalb des Querschnitts des Führungsteils 7 befindet. Durch Druckmittelzufuhr aus der Spritze 11 kann der Hohlkörper 12 in eine Expansionsstellung aufgebläht werden, in welcher er sich an die Wandung des Markraums 2 anlegt und damit dessen proximalen Abschnitt 2a gegen den distalen Abschnitt 2b abdichtet. Der Führungsteil 7 kann darstellungsgemäss mit einem Absperrorgan 13 versehen sein, welches einerseits eine rücklaufsichere Zufuhr des durch die Spritze 11 eingebrachten Druckmittels zum Hohlkörper 12 gestattet und andererseits zwischen einer eine Abfuhr des Druckmittels verhindernden Sperrstellung und einer diese Abfuhr zulassenden Lösestellung verstellbar ist, wobei das abgeführte Druckmittel zweckmässigerweise wieder in die Spritze 13 zurückgeführt wird.

Der Führungsteil 7 und die Entlastungsleitungen 8 und 9 können zu einer miteinander verbundenen Handhabungseinheit zusammengefasst oder, wie in der Zeichnung dargestellt, voneinander unabhängig, je für sich einführbar und zurückziehbar angeordnet sein. Dabei sind die Entlastungsleitungen 8 und 9 so ausgeführt und angeordnet, dass die eine, erste Entlastungsleitung 8 in der Betriebsstellung mit ihrem die Oeffnung 8a enthaltenden Endabschnitt über den mit dem Hohlkörper 12 verbundenen Endabschnitt des Führungsteils 7 vorsteht und damit eine Verbindung des distalen Endabschnitts 2b des Markraums 2 nach aussen herstellt, während die zweite Entlastungsleitung 9 mit ihrem die Oeffnung 9a enthaltenden Endabschnitt in Einführrichtung vor dem mit dem Hohlkörper 12 verbundenen Endabschnitt des Führungsteils 7 endet und damit eine Verbindung des Bodenbereichs des proximalen Abschnitts 2a nach aussen ermöglicht.

Der Führungsteil 7 und die Entlastungsleitungen 8 und 9 können auch auf andere Weise, z.B. in Form von in einem gemeinsamen Bandkörper ausgebildeten, diesen über seine Länge durchsetzenden Kanälen ausgeführt sein, von denen einer mit dem Hohlkörper 12 verbunden ist.

Vor dem Einsetzen des Verankerungsschafts 4 in den zu seiner Aufnahme vorbereiteten Markraum 2 werden der Führungsteil 7 und die Entlastungsleitungen 8 und 9 der Vorrichtung 6 bis auf eine vorbestimmte, für die Aufnahme des Verankerungsschafts 4 erforderliche Tiefe in den Markraum 2 eingeführt. Das äussere Ende des Führungsteils 7 kann mit der Spritze 11 fest verbunden oder an diese, wie in Fig.1 angedeutet, über eine lösbare Kupplung 14 angeschlossen sein. Anstelle des dargestellten Absperrorgans 13 kann auch eine andere Ausführung, z.B. eine in die Spritze 11 - oder in die Kupplung 14 - integrierte Absperranordnung, vorgesehen sein.

Zum Anbringen der Markraumsperre 5 wird die mit Druckmittel gefüllte Spritze 11 an den Führungsteil 7 angesetzt, worauf durch Druckmittelzufuhr in den Führungsteil 7 der Hohlkörper 12 aus seiner im Bereich des Querschnitts des Führungsteils 7 befindlichen Grundstellung in die den Markraum 2 unterteilende Expansionsstellung aufgebläht wird, in welcher er den proximalen Abschnitt 2a des Markraums 2 gegen dessen distalen Abschnitt 2b dichtend abschliesst. Durch eine in die Operationsöffnung 3 einführbare Austrittstülle 15 einer nicht weiter dargestellten Zuführvorrichtung wird Knochenzement in den Markraumabschnitt 2a bis auf einen Füllstand F eingefüllt, der um ein vorbestimmtes Mass A unterhalb der Operationsöffnung 3 liegt. Beim Einfüllen des Knochenzements kann im Bodenbereich des Markraumabschnitts 2a befindliche Luft und/oder Flüssigkeit durch die unmittelbar oberhalb des Hohlkörpers 12 positionierbare Oeffnung 9a in die Entlastungsleitung 9 entweichen und aus dieser abgeleitet, gegebenenfalls abgesaugt werden.

Nach dem Einfüllen des Knochenzements wird der zu implantierende Verankerungsschaft 4 teilweise, mit einem ausserhalb der Operationsöffnung 3 verbleibenden Ueberstand gegenüber der dargestellten Stellung, in den noch weichen Knochenzement eingeführt, wobei der im Bereich des Abstands A bestehende, freie obere Endbereich 16 des Markraumabschnitts 2a durch den verdrängten Knochenzement aufgefüllt wird. Durch die unterhalb des Hohlkörpers 12 befindliche Oeffnung 8a der Entlastungsleitung 8 kann aus dem proximalen Abschnitt 2a verdrängte Luft und/oder Flüssigkeit aus dem distalen Abschnitt 2b des Markraums 2 abgeleitet, gegebenenfalls abgesaugt werden. Entsprechend kann auch im Falle einer durch das Einbringen des Knochenzements und/oder das Einsetzen des Verankerungsschafts 4 bedingten Luftzufuhr aus dem proximalen Abschnitt 2a eine Erhöhung des im distalen Markraumabschnitt 2b herrschenden Drucks verhindert werden, wodurch die Gefahr der Bildung einer durch Ueberdruck verursachten Fettembolie aus dem distalen Markraumbereich vermieden wird.

Nach teilweiser Aushärtung des Knochenzements kann nach entsprechender Betätigung des Absperrorgans 13 das Druckmittel in den Zylinder der Spritze 11 aufgezogen und damit der Hohlkörper 12 aus seiner Expansionsstellung in die im Querschnittsbereich des Führungsteils 7 befindliche Grundstellung geschrumpft werden, in welcher er, zusammen mit dem Führungsteil 7, ohne wesentliche Verwerfung oder anderweitige Beschädigung des vorgängig gebildeten Zementzylinders aus diesem herausgezogen werden kann. Die Entlastungsleitungen 8 und 9 können gemeinsam mit dem Führungsteil 7 oder von diesem unabhängig aus dem Markraum 2 herausgezogen werden.

Nach dem Entfernen des Führungsteils 7 und der Entlastungsleitungen 8 und 9 kann der Verankerungsschaft 4 vollständig in die in Fig.1 dargestellte Position im noch nicht voll ausgehärteten Knochenzementbett eingepresst werden, wobei die durch den Führungsteil 7 und die Entlastungsleitungen 8 und 9 gebildeten Kanäle im Zementzylinder durch den verdrängten Knochenzement geschlossen bzw. aufgefüllt werden. Ein allfällig verbleibender Teil des beim vollständigen Einführen des Verankerungsschafts 4 verdrängten Knochenzements kann im oberen Endbereich 16 des Markraums 2 aufgenommen werden, da der Aushärtungsgrad des Knochenzements von der tiefsten Stelle des Markraumabschnitts 2a gegen dessen oberen Endbereich 16 hin abnimmt.

Die beschriebene Vorrichtung 6 gestattet die Anbringung einer temporär einsetzbaren Markraumsperre 5, welche auf einfache Weise, innerhalb weniger Sekunden, durch Aufblähen positioniert und nach dem Aushärten des Knochenzements auf ebenso einfache Weise, durch Schrumpfen in die Grundstellung, in den Querschnittsbereich des Führungsteils 7 zurückgeführt und hierauf aus dem Markraum 2 entfernt werden kann. Ein wesentlicher Vorteil der beschriebenen Markraumsperre 5 besteht darin, dass sie an Markräume mit unterschiedlichen Querschnittsabmessungen angepasst werden kann, wobei die Markräume nicht ausgemessen werden müssen. Insbesondere müssen weder Sperrelemente verschiedener Grösse noch spezielle Implantationsinstrumente vorrätig gehalten werden.

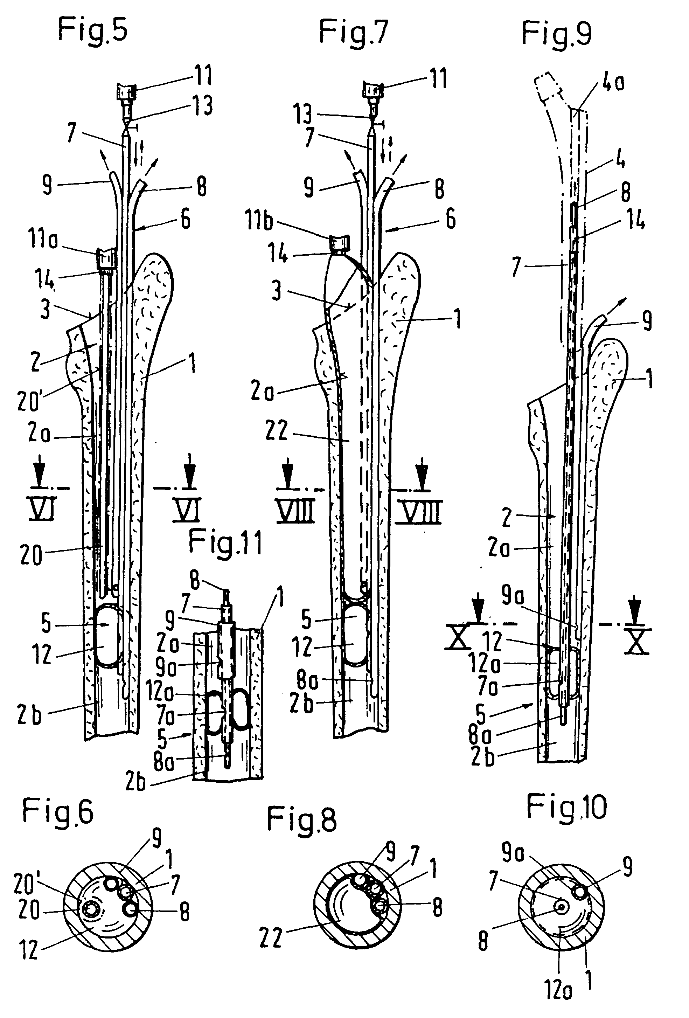

Die Vorrichtung 6 nach den Fig.5 und 6 entspricht im wesentlichen derjenigen nach den Fig.1 bis 4, wobei zusätzlich ein in den Markraum 2 einführbarer rohrförmiger Verdrängungskörper 20 zum Verdichten des eingebrachten Knochenzements vorgesehen ist. Der Verdrängungskörper 20 kann darstellungsgemäss durch einen quer zu seiner Längserstreckung expandierbaren Schlauchteil gebildet sein, der an dem in den Markraum 2 einführbaren Ende verschlossen ist und der mit seinem aus dem Markraum 20 vorstehenden Ende an die Spritze 11 oder an eine weitere Quelle eines Druckmittels, darstellungsgemäss eine entsprechende Spritze 11a, anschliessbar ist. Vor oder nach dem Einführen des Verankerungsschafts 4 in den Markraum 2, vorzugsweise nach dem Entfernen des Führungsteils 7 und der Entlastungsleitungen 8 und 9 aus dem teilweise ausgehärteten Zementzylinder, kann durch Druckmittelzufuhr aus der Spritze 11a der Verdrängungskörper 20 aus einer mit vollen Linien dargestellten Grundstellung in eine strichpunktiert angedeutete Expansionsstellung 20' aufgebläht werden, um den eingebrachten Knochenzement zu verdichten und die im Bereich des Führungsteils 7 und der Entlastungsleitungen 8 und 9 hinterlassenen Hohlräume auszufüllen. Durch Zurückführen des Druckmittels in die Spritze 11a kann der Verdrängungskörper 20 in die Grundstellung geschrumpft werden, in welcher er aus dem Knochenzementbett entfernt werden kann. Der im Bereich des entfernten Verdrängungskörpers 20 verbleibende Hohlraum kann durch den beim vollständigen Eindrücken des Verankerungsschafts 4 verdrängten Knochenzement oder durch frisch zugeführten Knochenzement ausgefüllt werden.

Bei der Ausführung nach den Fig.7 und 8 enthält die Vorrichtung 6 zusätzlich eine in den proximalen Abschnitt 2b des Markraums 2 einführbare, elastisch verformbare längliche Hülle 22, deren in den Markraum 2 einführbares Ende verschlossen ist und deren ausserhalb des Markraums 2 verbleibendes Ende ebenfalls an eine Druckmittelquelle, darstellungsgemäss an eine entsprechende Spritze 11b, anschliessbar ist. Die Hülle 22 ist zum temporären Auskleiden des für die Implantation vorbereiteten Markraumabschnitts 2b bestimmt. Dadurch kann insbesondere während der Zeit der Zementvorbereitung eine auf einfache Weise einsetzbare und entfernbare proximale Markraumtamponade erhalten werden, welche - anstelle einer üblichen, durch eine Gaze-Fahne gebildeten Tamponade - zur Blutstillung verwendbar ist. Die Hülle 22 kann durch Druckmittelzufuhr aufgebläht und an die Wandung des Markraumabschnitts 2a sowie an den Führungsteil 7 und die Entlastungsleitungen 8 und 9 angepresst und vor dem Einbringen des Knochenzements durch Druckmittelabfuhr geschrumpft und aus dem Markraumabschnitt 2a herausgezogen werden. Die Hülle 22 kann ebenso vor dem Einführen der Vorrichtung 6 in den Markraum 2 eingeführt und aufgebläht werden, um diesen vor dem Anbringen der Markraumsperre 5 abzuschliessen.

Entsprechend der Darstellung nach den Fig.9 und 10 kann als Führungsteil 7 ein formstabiles, z.B. metallisches Rohr vorgesehen sein, dessen in den Markraum 2 einführbarer, in axialer Richtung verschlossener Endabschnitt von dem bei dieser Ausführung in Form eines Ringes 12a ausgebildeten Hohlkörper 12 umgeben ist, der den Führungsteil 7 in der druckentlasteten Grundstellung eng umschliesst und der durch Druckmittelzufuhr radial in eine Expansionsstellung aufblähbar ist, in welcher er den Endabschnitt des Führungsteils 7 reifenartig umgibt und den Markraumabschnitt 2a abschliesst. Der Führungsteil 7, dessen aus dem Markraum 2 vorstehender Endabschnitt durch die Kupplung 14 verschliessbar und über diese an die Druckmittelquelle anschliessbar ist, wird bei dieser Ausführung somit durch den aufgeblähten Hohlkörper 12 innerhalb des Markraums 2 zentriert und ist als Führungs- und Zentrierelement für den in den Markraum 2 einzusetzenden Verankerungsschaft 4 verwendbar.

Die Entlastungsleitung 8 kann vom Führungsteil 7 getrennt angeordnet oder, wie dargestellt, in einer teleskopartigen Anordnung durch ein den Führungsteil 7 im wesentlichen koaxial durchsetzendes Rohr gebildet sein. Dieses ist gegen den vom Hohlkörper 12 umgebenen Endabschnitt des Führungsteils 7 abgedichtet und steht mit seinem die Öffnung 8a enthaltenden Ende in den Markraumabschnitt 2b vor. Der Verankerungsschaft 4 ist darstellungsgemäss mit einer über seine Länge durchgehenden, an die Dicke des Führungsteils 7 angepassten Bohrung 4a ausgeführt und kann bei der Implantation auf den annähernd in der fiktiven Mittelachse des Markraums 2 positionierbaren, neigbar gehaltenen Führungsteil 7 aufgesteckt und in den mit Knochenzement gefüllten Markraumabschnitt 2a eingeschoben werden.

Nach teilweiser Aushärtung des Knochenzements kann der Führungsteil 7 über die Kupplung 14 an die Spritze 11 angeschlossen, das Druckmittel in den Zylinder der Spritze 11 zurückgeführt und damit der Hohlkörper 12 in die Grundstellung geschrumpft werden, in welcher er, zusammen mit dem Führungsteil 7 und der Entlastungsleitung 8 durch die Bohrung 4a des Verankerungsschafts 4 herausgezogen werden kann. Hierauf kann die Entlastungsleitung 9 in bereits beschriebener Weise aus dem Knochenzementbett herausgezogen werden. Bei dieser Ausführung kann sich die Wand des expandierten Hohlkörpers 12 mit ihrem ganzen Umfang an die Wandung des Markraums 2 anlegen, so dass die Bildung von entlang dieser Wandung verlaufenden Durchtrittsöffnungen verhindert wird und damit eine sichere Abdichtung zwischen den Markraumabschnitten 2a und 2b erzielbar ist. Zudem wird, im Vergleich zu den vorstehend beschriebenen Ausführungen, die Anzahl der nach dem Entfernen der Markraumsperre 5 entlang der Wandung des proximalen Abschnitts 2a verbleibenden, durch Verdichten des Knochenzements auszufüllenden Hohlräume, darstellungsgemäss auf einen durch die Entlastungsleitung 9 gebildeten, einzigen Kanal verringert.

Mindestens eine der Entlastungsleitungen 8 und 9 kann auch durch ein formstabiles, z.B. metallisches Rohr gebildet sein. Nach einer abgewandelten, nicht dargestellten Ausführungsform der Erfindung können die Entlastungsleitungen 8 und 9 ebenfalls teleskopartig angeordnet sein. Gemäss Fig 11 kann die Entlastungsleitung 9 durch ein den Führungsteil 7 umgebendes, im Bodenbereich des Markraumabschnitts 2a offenes Entlastungsrohr gebildet sein, welches vor dem Aufsetzen des Verankerungsschafts 4 auf den Führungsteil 7 aus dem von Hohlräumen im Umfangsbereich freien Knochenzementbett entfernt oder, bei entsprechend grossem Querschnitt der Bohrung 4a, zusammen mit dem Führungsteil 7 aus dem eingesetzten Verankerungsschaft 4 herausgezogen werden kann. Es ist auch eine Ausführung möglich, bei der auf die Anordnung der oder einer der Entlastungsleitungen 8, 9 verzichtet wird. Die erfindungsgemässe Vorrichtung ist auch für die Anbringung von Markraumsperren in anderen Implantationsbereichen, z.B. für Knie- oder Ellbogenprothesen, geeignet.

Zusammenfassend lässt sich die Erfindung wie folgt beschreiben:

Ein in den Markraum 2 einführbarer, rohrförmiger Führungsteil 7 ist an eine Druckmittelquelle 11 angeschlossen und mit einem Sperrelement in Form eines ballonartig aufblähbaren Hohlkörpers 12 verbunden. Der Hohlkörper 12 wird durch Druckmittelzufuhr aus einer im Querschnittsbereich des Führungsteils 7 befindlichen Grundstellung in eine Expansionsstellung aufgebläht, in welcher er einen mit Knochenzement zu füllenden, einen Prothesenschaft 4 aufnehmenden Markraumabschnitt 2a gegen den benachbarten Markraumabschnitt 2b verschliesst. Nach dem Einsetzen eines Teils des Prothesenschafts 4 in das Knochenzementbett kann der Hohlkörper 12 durch Druckmittelabfuhr in die Grundstellung geschrumpft und mit dem Führungsteil 7 aus dem Markraum 2 zurückgezogen werden. Der dabei im Knochenzementbett entstehende Hohlraum wird durch beim Einführen des restlichen Abschnitts des Prothesenschafts 4 verdrängten Knochenzement ausgefüllt. Auf diese Weise wird eine temporär anbringbare und vor dem endgültigen Aushärten des Knochenzements aus dem Markraum 2 entfernbare Markraumsperre 5 erhalten.

## Patentansprüche

1. Vorrichtung zum Anbringen einer Markraumsperre (5) im Markraum (2) eines Röhrenknochens, der zur Aufnahme eines unter Verwendung von Knochenzement zu implantierenden Verankerungsschafts (4) einer Endoprothese bestimmt ist, mit einem in den Markraum (2) einführbaren, rohrförmigen Führungsteil (7), der mit einem im Markraum (2) positionierbaren, elastisch verformbaren Sperrelement in Form eines ballonartig expandierbaren Hohlkörpers (12) verbunden ist, welcher an eine ausserhalb des Markraums (2) angeordnete Quelle (11) eines Druckmittels anschliessbar ist und welcher zwischen einer bei druckentlastetem Zustand innerhalb des Querschnitts des Führungsteils (7) befindlichen Grundstellung und einer bei Druckmittelzufuhr einnehmbaren, den Querschnitt des Markraums (2) abschliessenden Expansionsstellung verstellbar ist, **dadurch gekennzeichnet,** dass die Vorrichtung eine von dem Verankerungsschaft (4) der zu implantierenden Endoprothese verschiedene, separate Vorrichtung ist, welche nach dem Einbringen von Knochenzement in den Markraum wieder aus dem Markraum entfernbar ist, und dass das elastisch verformbare Sperrelement in Form des ballonartig expandierbaren Hohlkörpers (12) kommunizierend mit dem rohrförmigen Führungsteil (7) verbunden ist.

2. Vorrichtung nach Anspruch 1, **dadurch gekennzeichnet**, dass der Führungsteil (7) mit einem Absperrorgan (13, 14) verbunden ist, welches zwischen einer eine Abfuhr des Druckmittels sperrenden Sperrstellung und einer die Abfuhr zulassenden Lösestellung verstellbar ist.

3. Vorrichtung nach Anspruch 1 oder 2, **dadurch gekennzeichnet**, dass dem Führungsteil (7) eine in den Markraum (2) einführbare und zurückziehbare, rohrförmige Entlastungsleitung (8) zugeordnet ist, die eine in Einführrichtung hinter dem mit dem Hohlkörper (12) verbundenen Abschnitt des Führungsteils (7) positionierbare Eintrittsöffnung (8a) für Luft und/oder Flüssigkeit aufweist.

4. Vorrichtung nach einem der vorangehenden Ansprüche, **dadurch gekennzeichnet**, dass dem Führungsteil (7) eine in den Markraum (2) einführbare und zurückziehbare, zweite rohrförmige Entlastungsleitung (9) zugeordnet ist, die eine in Einführrichtung vor dem mit dem Hohlkörper (12) verbundenen Abschnitt des Führungsteils (7) positionierbare Eintrittsöffnung (9a) für Luft und/oder Flüssigkeit aufweist.

5. Vorrichtung nach einem der vorangehenden Ansprüche, gekennzeichnet durch einen in den Markraum (2) einführbaren, länglichen Verdrängungskörper (20) in Form eines gegen den Hohlkörper (12) hin verschlossenen, quer zu seiner Längserstreckung expandierbaren Schlauchteils, welcher an die oder eine weitere Quelle (11a) eines Druckmittels anschliessbar ist und welcher durch Druckmittelzufuhr aus einer Grundstellung in eine zum Verdichten des in den Markraum (2) eingebrachten Knochenzements bestimmte Expansionsstellung (20') aufblähbar ist.

6. Vorrichtung nach einem der vorangehenden Ansprüche, **dadurch gekennzeichnet**, dass der Führungsteil (7) als formstabiles, in den Markraum (2) freistehend einsetzbares Zentrierelement ausgebildet ist, welches zum Zusammenführen mit einer den Verankerungsschaft (4) in Richtung seiner Längsachse durchsetzenden Bohrung (4a) bestimmt ist, und dass der Hohlkörper (12) in Form eines den Führungsteil (4) umgebenden, radial expandierbaren Ringes (12a) ausgeführt ist (Fig.9).

7. Vorrichtung nach einem der Ansprüche 3 bis 6, **dadurch gekennzeichnet**, dass der Führungsteil (7) und mindestens eine der Entlastungsleitungen (8,9) zu einer Handhabungseinheit zusammengefasst sind.

8. Vorrichtung nach einem der Ansprüche 3 bis 7, **dadurch gekennzeichnet**; dass der Führungsteil (7) und mindestens einer der Entlastungsleitungen (8,9) teleskopartig, im wesentlichen koaxial zueinander angeordnet sind.

9. Vorrichtung nach einem der Ansprüche 1 bis 5, gekennzeichnet durch eine in den Markraum (2) einführbare, gegen den Hohlkörper (12) hin verschlossene, an die oder eine weitere Quelle (11b) eines Druckmittels anschliessbare, elastisch verformbare Hülle (22) zum temporären Auskleiden des Markraums (2), die durch Druckmittelzufuhr an die Wandung des Markraums anpressbar ist.

## Claims

1. Apparatus for the placement of a medullary space blocker (5) in the medullary space (2) of a tubular bone intended to receive an-anchoring shaft (4) of an endoprosthesis which is to be implanted with the use of bone cement, comprising a tubular guide part (7) which can be inserted into the medullary space (2), wherein the tubular guide part (7) is connected to an elastically deformable blocking element-which is positionable in the medullary space (2), which has the form of a hollow body (12) expansible in the manner of a balloon, which is connectable outside of the medullary space (2) to a source (11) of a pressure medium, and which is adjustable between a basic position situated substantially inside the cross-section of the guide part (7) in a state relieved of pressure, and an expanded position in which the cross-section of the medullary space (2) is sealed off and which is adoptable on the supply of pressure medium **characterised in that** the apparatus is a separate apparatus different from the anchoring shaft (4) of the endoprosthesis to be implanted, which can be removed again from the medullary space after the introduction of bone cement into the medullary space and in that the elastically deformable blocking element in the form of the hollow body expansible in balloon-like manner is communicatingly connected to the tubular guide part (7).

2. Apparatus in accordance with claim 1, **characterized in that** the guide part (7) is connected to a closure member (13, 14) adjustable between a blocking position which blocks the removal of the pressure medium and a release position which permits the removal.

3. Apparatus in accordance with claim 1 or 2, **characterized in that** the guide part (7) is associated with a tubular relief line (8) which can be inserted into and withdrawn from the medullary space (2) and which has an inlet aperture (8a) for air and/or liquid positionable in the insertion direction behind the portion of the guide part (7) connected to the hollow body (12).

4. Apparatus in accordance with one of the preceding claims, **characterized in that** the guide part (7) is associated with a second tubular relief line (9) which can be inserted into and withdrawn from the medullary space (2) and which has an inlet opening (9a) for air and/or liquid positionable in the insertion direction in front of the portion of the guide part (7) connected to the hollow body (12).

5. Apparatus in accordance with one of the preceding claims, characterized by an elongate displacement body (20) which can be inserted into the medullary space (2) and which has the form of a hose part which is expansible transverse to its elongate extension, which is closed relative to the hollow body (12) and which is connectable to the source or a further source (11a) of a pressure medium and which can be inflated from a basic position into an expanded position (20') by the supply of the pressure medium to compress the bone cement introduced into the medullary space (2).

6. Apparatus in accordance with one of the preceding claims, **characterized in that** the guide part (7) is formed as a dimensionally stable centering element which can be inserted into the medullary space (2) so as to be free standing and which is intended to be brought together with a bore (4a) passing through the anchoring shaft (4) in the direction of its longitudinal axis, and in that the hollow body (12) is implemented in the form of a radially expansible ring (12a) surrounding the guide part (4) (Fig. 9).

7. Apparatus in accordance with one of the claims 3 to 6, **characterized in that** the guide part (7) and at least one of the relief lines (8, 9) are combined to form a handling unit.

8. Apparatus in accordance with one of the claims 3 to 7, **characterized in that** the guide part (7) and at least one of the relief lines (8, 9) are arranged telescopically substantially coaxially to one another.

9. Apparatus in accordance with one of the claims 1 to 5, characterized by an elastically deformable sheath (22) for temporarily lining the medullary space (2), wherein the sheath (22) is insertable into the medullary space (2), is closed relative to the hollow body (12) and is connectable to the source or to a further source (11b) of a pressure medium, and can be pressed against the wall of the medullary space by the supply of a pressure medium.

## Revendications

1. Dispositif pour poser un obturateur intramédullaire (5) dans le canal médullaire (2) d'un os tubulaire qui est destiné à recevoir une tige d'ancrage (4) d'une endoprothèse à implanter en utilisant du ciment à os, avec une pièce de guidage tubulaire (7) insérable dans le canal médullaire (2) qui est reliée à un élément d'obturation déformable élastiquement, pouvant être positionné dans le canal médullaire (2) sous la forme d'un corps creux (12) pouvant être expansé à la manière d'un ballon, qui peut être raccordé à une source (11) d'un milieu sous pression disposée à l'extérieur du canal médullaire (2) et qui est ajustable entre une position initiale se trouvant à l'état détendu à l'intérieur de la section transversale de la pièce de guidage (7) et une position d'expansion occupée lors d'une amenée de milieu sous pression, fermant la section transversale du canal médullaire (2), **caractérisé en ce que** le dispositif est un dispositif séparé, se différenciant de la tige d'ancrage (4) de l'endoprothèse à implanter qui, après l'introduction du ciment à os dans le canal médullaire, peut être retiré de nouveau du canal médullaire, et en ce que l'élément d'obturation déformable élastiquement est relié sous la forme d'un corps creux (12) expansible à la manière d'un ballon de façon à communiquer avec la pièce de guidage tubulaire (7).

2. Dispositif selon la revendication 1, **caractérisé en ce que** la pièce de guidage (7) est reliée à un organe d'obturation (13, 14) qui est déplaçable entre une position d'obturation bloquant l'évacuation du milieu sous pression et une position de relâchement permettant l'évacuation.

3. Dispositif selon la revendication 1 ou 2,
**caractérisé en ce qu**'il est associé à la pièce de guidage (7) un conduit de décharge tubulaire (8) insérable dans le canal médullaire (2) et retirable de celui-ci, qui présente une ouverture d'entrée (8a) pour de l'air et/ou du liquide pouvant être positionnée dans la direction d'insertion derrière le tronçon de la pièce de guidage (7) relié au corps creux (12).

4. Dispositif selon l'une des revendications précédentes, **caractérisé en ce qu**'il est associé à la pièce de guidage (7) un deuxième conduit de décharge tubulaire (9) insérable dans le canal médullaire (2) et retirable de celui-ci qui présente une ouverture d'entrée (9a) pour de l'air et/ou du liquide pouvant être positionnée dans la direction d'insertion devant le tronçon de la pièce de guidage (7) relié au corps creux (12).

5. Dispositif selon l'une des revendications précédentes, caractérisé par un corps de refoulement oblong (20) insérable dans le canal médullaire (2), sous la forme d'une partie tubulaire fermée vers le corps creux (12), expansible transversalement à son extension longitudinale, qui peut être raccordé à la ou une autre source (11a) d'un milieu sous pression et qui peut être gonflé, par une amenée de milieu sous pression, à partir d'une position d'initiale dans une position d'expansion (20') destinée au compactage du ciment à os introduit dans le canal médullaire (2).

6. Dispositif selon l'une des revendications précédentes, **caractérisé en ce que** la pièce de guidage (7) est réalisée comme élément de centrage d'une forme stable, pouvant être placée librement dans le canal médullaire (2) qui est destiné à être réuni avec un perçage (4a) traversant la tige d'ancrage (4) dans le sens de son axe longitudinal, et en ce que le corps creux (12) est réalisé sous la forme d'une bague (12a) expansible radialement, entourant la pièce de guidage (4) (Figure 9).

7. Dispositif selon l'une des revendications 3 à 6,
**caractérisé en ce que** la pièce de guidage (7) et au moins l'un des conduits de décharge (8, 9) sont réunis en une unité de manipulation.

8. Dispositif selon l'une des revendications 3 à 7,
**caractérisé en ce que** la pièce de guidage (7) et au moins l'un des conduits de décharge (8, 9) sont disposés d'une manière téléscopique, sensiblement coaxialement l'un relativement à l'autre.

9. Dispositif selon l'une des revendications 1 à 5,
caractérisé par une enveloppe (22) déformable élastiquement, insérable dans le canal médullaire (2), fermée vers le corps creux (12), pouvant être raccordée à la ou une source supplémentaire (11b) d'un milieu sous pression, pour revêtir temporairement le canal médullaire (2), qui peut être appliqué par l'amenée du milieu sous pression à la paroi du canal médullaire.
